# FASCICULE DE BREVET EUROPEEN

(11) **EP 1 715 064 B1**
(45) Date de publication et mention de la délivrance du brevet: **25.02.2009**
(21) Numéro de dépôt: 06013029.1
(22) Date de dépôt: 05.06.1990
(51) Int. Cl.: C12Q 1/70, C12Q 1/68, C12N 15/49

(54) **Séquences nucléotidiques issues du génome des rétrovirus du type HIV-1, HIV-2 et SIV, et leurs applications notamment pour l'amplification de séquences de pol de ces génomes de ces rétrovirus et pour le diagnostic in vitro des infections dûes à ces virus**
Nukleotid-Sequenzen von HIV-1, HIV-2 und SIV Retrovirusgenomen, ihre Verwendung zur Amplifizierung von pol-Sequenzen dieser Retroviren und zur In Vitro Diagnostik von diesen Viren verursachten Infektionen
Nucleotide sequences derived from the genomes of the retroviruses HIV-1, HIV-2 and SIV, and their application to the amplification of pol sequences in these viral genomes and to the in vitro diagnosis of infections resulting from these viruses

(30) Priorité: 02.06.1989 FR 8907354; 20.09.1989 FR 8912371
(43) Date de publication de la demande: 25.10.2006
(62) Demande divisionnaire de: 05014676.0
(73) Titulaire: INSTITUT PASTEUR, 75724 Paris (FR); INSTITUT NATIONAL DE LA SANTE ET DE LA RECHERCHE MEDICALE (INSERM), 75654 Paris Cédex 13 (FR)
(72) Inventeur: Moncany, Maurice, 75019 Paris (FR); Montagnier, Luc, 92350 Le Plessis-Robinson (FR)
(74) Mandataire: Desaix, Anne

(56) Documents cités:
- EP-A- 0 229 701
- EP-A- 0 253 701
- EP-A- 0 269 445
- EP-A- 0 269 520
- WO-A-86/02383
- WO-A-88/05440
- COUTLEE F ET AL: "IMMUNODETECTION OF DNA WITH BIOTINYLATED RNA PROBES: A STUDY OF REACTIVITY OF A MONOCLONAL ANTIBODY TO DNA-RNA HYBRIDS" ANALYTICAL BIOCHEMISTRY, ACADEMIC PRESS, NEW YORK, NY, US, vol. 181, no. 1, 15 août 1989 (1989-08-15), pages 96-105, XP008030273 ISSN: 0003-2697
- MACK D H ET AL: "A SENSITIVE METHOD FOR THE IDENTIFICATION OF UNCHARACTERIZED VIRUSES RELATED TO KNOWN VIRUS GROUP HEPADNAVIRUS MODEL SYSTEM" PROCEEDINGS OF THE NATIONAL ACADEMY OF SCIENCES OF THE UNITED STATES, NATIONAL ACADEMY OF SCIENCE, WASHINGTON, DC, US, vol. 85, no. 18, 1988, pages 6977-6981, XP002148107 ISSN: 0027-8424
- WAIN-HOBSON S ET AL: "NUCLEOTIDE SEQUENCE OF THE AIDS VIRUS, LAV" CELL, MIT PRESS, CAMBRIDGE, MA,, US, vol. 40, no. 1, 1985, pages 9-17, XP000608742 ISSN: 0092-8674
- DONEHOWER L A ET AL: "THE USE OF PRIMERS FROM HIGHLY CONSERVED POL REGIONS TO IDENTIFY UNCHARACTERIZED RETROVIRUSES BY THE POLYMERASE CHAIN REACTION" JOURNAL OF VIROLOGICAL METHODS, AMSTERDAM, NL, vol. 28, no. 1, avril 1990 (1990-04), pages 33-46, XP000407104 ISSN: 0166-0934

## Description

La présente invention est relative à des séquences oligonucléotidiques utilisables pour la mise en oeuvre de techniques d'amplification de séquences nucléiques spécifiques de rétrovirus d'immunodéficience humaine du type HIV ou de rétrovirus d'immunodéficience du singe du type SIV.

L'invention concerne en particulier l'application de ces séquences à des méthodes de diagnostic in vitro chez l'homme de l'infection d'un individu par un rétrovirus du type HIV (actuellement HIV-1 et/ou HIV-2).

L'isolement et la caractérisation de rétrovirus regroupés sous les désignations HIV-1 et HIV-2 ont été décrits dans les demandes de brevet européen EP201540 et EP239425 respectivement. Ces rétrovirus ont été isolés chez plusieurs malades présentant des symptômes d'une lymphadénopathie ou d'un Syndrome d'Immunodeficience Acquise (SIDA).

Les rétrovirus du type HIV-2 comme les rétrovirus du type HIV-1, se caractérisent par un tropisme pour les lymphocytes T4 humains et par un effet cytopathogène à l'égard de ces lymphocytes lorsqu'ils s'y multiplient, pour alors causer entre autres des polyadénopathies généralisées et persistantes, ou un SIDA.

Un autre rétrovirus, dénommé SIV-1, cette dénomination remplaçant la dénomination antérieurement connue STLV-III, a été isolé chez le singe macaque rhésus (M.D. DANIEL et al. Science, 228, 1201 (1985) ; N.L. LETWIN et al, Science, 230, 71 (1985) sous l'appellation "STLV-IIImac").

Un autre rétrovirus, désigné "STLV-III_{AGM}", (ou SIV_{AGM}) a été isolé chez des singes verts sauvages. Mais contrairement aux virus présents chez le singe macaque rhésus, la présence de STLV-III_{AGM} ne semble pas induire une maladie du type SIDA chez le singe vert d'Afrique.

Pour la commodité du langage, ces virus ne seront plus désignés dans ce qui suit que par l'expression SIV (l'expression SIV est l'abréviation anglaise de "Simian Immunodeficency Virus" (Virus d'immunodéficience du singe) éventuellement suivie d'une abréviation désignant l'espèce de singe dont ils sont issus par exemple "MAC" pour le macaque" ou "AGM" pour le singe vert d'Afrique (abréviation de "African Green Monkey").

Une souche du rétrovirus SIV-1Mac à été déposée à la C.N.C.M le 7 février 1986 sous le n° I-521.

La poursuite de l'étude des rétrovirus HIV-1 et HIV-2 a également conduit à l'obtention de séquences d'ADN complémentaires (ADNc) des ARN de leur génome. La séquence nucléotidique complète d'un ADNc d'un rétrovirus représentatif de la classe HIV-2 (HIV-2 ROD) a été déposée le 21/02/1986 à la C.N.C.M. sous le n° I-522, sous le nom de référence LAV-2 ROD.

De même, la séquence nucléotidique complète d'un ADNc d'un rétrovirus représentatif de la classe HIV-1 est décrite par WAIN HOBSON, SONIGO, COLE, DANOS et ALIZON dans CE11 (janvier 1985).

Egalement pour la commodité du langage, les virus du type HIV-1 et HIV-2 seront parfois désignés dans ce qui suit par l'expression HIV.

Les méthodes de diagnostic in vitro des infections par des virus du type HIV-1 ou HIV-2 existant actuellement, font appel à la détection d'anticorps ANTI-HIV-1 ou anti-HIV-2 éventuellement présents dans un prélèvement biologique (biopsie) ou dans un fluide biologique, par exemple dans un sérum obtenu, à partir du patient à l'étude, par mise en contact de ce fluide biologique avec des extraits ou antigènes d'HIV-1 ou d'HIV-2, dans des conditions permettant la production d'une réaction immunologique éventuelle de ces ces extraits ou antigènes avec ces anticorps.

De telles méthodes de diagnostic risquent d'être faussement négatives, en particulier dans le cas d'une infection récente d'un individu par les virus du type HIV.

Les techniques d'amplification génique sont d'un appoint considérable pour la mise au point de méthodes de diagnostic in vitro particulièrement sensibles de maladies virales. Parmi ces techniques d'amplification génique, on peut citer la technique PCR (Polymérase Chain Reaction) telle que décrite dans les demandes de brevet européen EP 200362 et EP 229701, ou encore la technique dite "Qβreplicase" décrite dans Biotechnology, vol.6, page 1197 (octobre 1988) et celle procédant à l'aide d'une ARN polymérase (T7RNA polymérase) décrite dans la demande de brevet international n° WO89/01050. Ces techniques permettent d'améliorer la sensibilité de détection des acides nucléiques des virus, et nécessitent l'utilisation d'amorces de synthèse spécifiques.

Afin d'amplifier à l'aide d'une technique d'amplification génique les gènes codant pour la transcriptase inverse des hépadnavirus HBV, WHV, GSHV et DHBV, Mack et al. (1985 ; PNAS. 85 : 6977-6981) propose des mélanges constitués d'un grand nombre d'isomères oligonucléotidiques dont les séquences ont été façonnées de façon non seulement à couvrir les différences de nucléotides existant entre ces différents hépadnavirus, mais également à tenir compte de la dégénérescence du code génétique.

Pour la recherche des virus du type HIV, le choix des amorces est problématique. En effet, du fait de la grande variabilité des séquences de nucléotides du génome viral, une amorce conforme à la séquence connue d'un isolat donné d'un virus du type HIV peut faillir à l'amplification de certains variants viraux du type HIV. D'autre part, même si une amorce est choisie dans une région conservée du génome d'un virus HIV à un autre, son "bon fonctionnement" n'est pas pour autant assuré et peut donner lieu à de mauvais rendements d'amplification.

La présente invention fournit précisément des amorces oligonucléotidiques permettant, entre autres, l'amplification, notamment à des fins diagnostiques, du génome de tous virus du type HIV et SIV, avec des rendements considérés comme maximum dans l'état actuel de la technique et surtout évitant la présence de nombreuses bandes aspécifiques.

Les amorces de la présente invention sont à la fois spécifiques des virus du groupe HIV-1 et des virus des groupes HIV-2 et SIV, et sont insensibles aux variations du génome de ces virus.

La présente invention a pour objet des amorces oligonueléotidiques, d'environ 15 à 30 nucléotides, utilisables pour l'amplification génomique des virus du type HIV-1 et/ou du type HIV-2 et SIV.

La demande décrit toute séquence nucléotidique caractérisée en ce que sa séquence :
- soit est choisie parmi celles qui sont contenues dans l'une des séquences nucléotidiques comprises dans les gènes gag, vpr et pol des virus HIV-1 Bru, HIV-1 Mal, HIV-1 Eli, HIV-2 ROD et SIV MAC, ou dans les gènes nef2, vif2 et vpx des virus HIV-2 ROD, et SIV MAC, ou dans les gènes env, nef1, vif1 et vpr des virus HIV-1 Bru, HIV-1 Mal, et HIV-1 Eli, et plus particulièrement parmi celles qui sont contenues dans les enchaînements nucléotidiques définis ci-après,
- soit (notamment pour les séquences les plus longues) contient l'une des séquences nucléotidiques susdites issues de HIV-1 Bru, ou HIV-1 Mal, ou HIV-1 Eli ou HIV-2 ROD ou SIVMac , ou contient une séquence nucléotidique complémentaire de l'une de ces dernières séquences, étant entendu que les nucléotides supplémentaires éventuels qui "débordent" la séquence nucléotidique du genre en question, du côté des extrémités 3' ou 5', coïncident de préférence avec ceux qui se trouvent placés en deçà des extrémités 5' ou 3' correspondant au sein même de la séquence complète des virus du type HIV-1, HIV-2 ou de SIV MAC, sus-mentionnés,
- soit, si cette séquence nucléotidique n'est pas identique à l'une des séquences nucléotidiques susdites, ou n'est pas complémentaire de l'une de ces séquences, est néanmoins susceptible de s'hybrider avec une séquence nucléotidique issue des virus HIV-1 Bru,HIV-1 Mal, HIV-1 Eli, et/ou avec une séquence nucléotidique issue du virus HIV-2 ROD ou SIV MAC sus-mentionnée. L'hybridation peut s'effectuer à une température de 60°C ± 1°C (de préférence 60°C ± 0,5°C), préconisée pour un optimum de rendement.

La numérotation des nucléotides mentionnés ci-dessous correspond à celle utilisée dans le manuel de référence "Human Retrovirus and AIDS-1989" édité par le "Los Alamos National Laboratory- New Mexico - USA".

(Les séquences des virus HIV-1 Mal, HIV-1 Eli ont été décrites par MONTAGNIER, SONIGO, WAIN-HOBSON et ALIZON dans la demande de brevet européen n° 86.401380 du 23/06/86).

Les séquences sont synthétisées sur synthétiseur commercialisé par Applied Biosystems (méthode phosphoro-amidites, ou sur tout autre appareil utilisant une méthode semblable.

La demande concerne plus particulièrement les séquences oligonucléotidiques caractérisées par les enchaînements nucléotidiques suivants (représentés dans le sens 5' → 3'; les initiales "S" et "AS" indiquent si l'oligonucléotide est sens ou antisens, c'est-à-dire si l'oligonucléotide est orienté respectivement dans le sens 5'O→ 3' ou dans le sens 3' O→ 5') :
1°) séquences communes aux génomes des virus HIV-1, HIV-2 et SIV (les séries de chiffres espacées d'un trait indiquent la position des nucléotides sur les génomes correspondant respectivement aux virus HIV-1 Bru, HIV-1 Mal, HIV-1 Eli, HIV-2 ROD et SIV) :
   . séquences spécifiques du gène gag du génome des virus sus-mentionnés (gène codant pour un groupe d'antigènes spécifiques du nucléoïde de ces virus).
      Certaines variantes peuvent être apportées sur certaines positions des séquences nucléotidiques indiquées ci-dessous, sans que les propriétés d'hybridation de ces séquences nucléotidiques avec les gènes des virus du type HIV et/ou SIV soient affectées. Les séquences nucléotidiques comportant ces variantes sont représentées en-dessous des séquences nucléotidiques initiales dont elles dérivent par remplacement d'une ou plusieurs bases. Les bases modifiées par rapport à celles des séquences nucléotidiques initiales sont indiquées en toute lettre à la verticale des positions correspondant aux bases qui ont été remplacées dans ces séquences initiales ; tandis que les bases des séquences initiales qui n'ont pas été remplacées dans les séquences comportant ces variantes sont indiquées à l'aide de pointillés.
      La synthèse des amorces se fait en utilisant toutes les variantes simultanément. C'est le mélange de toutes les variantes pour une séquence donnée qui est utilisé dans les tests. 
MMy1 :   TGG CGC CCG AAC AGG GAC
         ... ... .T. ... ... ...
         S, 636-653, 635-652, 636-653, 859-876, 834-851
 
MMy2 :    GGC CAG GGG GAA AGA AAA A
          ... .C. .C. ... ... ... .
          ... ... .A. ... ... ... .
          S,854-872,864-888,848-872,1160-1184, 1124-1148
 
MMy3 :    TGC CCA TAC AAA ATG TTT TA
          ... ... C.. T.T ... ... ..
       AS,900-881,916-897,900-881,1212-1193,1176-1157
 
MMy4 : TGC ATG GCT GCT TGA TG
       ... ..A ... ..C ..G ..
       AS,1385-1369,1419-1403,1385-1369,1703-1687,1667-1651
 
MMy4B : CTT TGC ATG GCT GCT TGA TG
..C ... ..A ... ..C ..G ..
AS, 1388-1369, 1421-1403, 1388-1369, 1706-1687,
1670-1651,
 
MMy4Bbis : CAT CAA GCA GCC ATG CAA AG
..C ..G ... ..T ... ..G ..
S, 1369-1388, 1403-1421, 1369-1388,
1687-1706, 1651-1670,
 
MMy28 : AGG GCT GTT GGA AAT GTG G -
... ... ... ... ..G ... .
S, 2021-2039, 2055-2073, 2024-2042, 2329-2349,
2299-2318,
 
MMy28bis : CCA CAT TTC CAG CAT CCC T
... ... ... ... ..G ... .
... ... ... ... ..C ... .
AS, 2039-2021, 2073-2055, 2042-2024, 2349-2329,
2318-2299
   . séquences spécifiques du gène vpr 
MMy18 : GAT AGA TGG AAC AAG CCC CAG
S, 5590-5610, 5585-5605, 5554-5574, 6233-6296,
6147-6170,
 
MMy19 : TCC ATT TCT TGC TCT CCT CTG T
AS, 5870-5849, 5865-5844, 5834-5813.
6551-6531, 6454-6431,
   . séquences spécifiques du gène pol : 
MMy29 : TAA AGC CAG GAA TGG ATG GCC CAA
... ... ... ... ... ... .A. ...
S, 2620-2643, 2615-2638, 2584-2607, 2971-2994,
2887-3010
 
MMy29bis : TTG GGC CAT CCA TTC CTG GCT TTA
... .T. ... ... ... ... ... ...
AS, 2643-2620, 2638-2615, 2607-2584, 2994-2971,
3010-2887,
 
MMy30 : TGG ACT GTC AAT GAC ATA CAG AA
... ... ... ... ..T ... ... ..
S, 3339-3361, 3334-3356, 3303-3325, 3690-3712,
3606-3628,
 
MMy30bis : TTC TGT ATG TCA TTG ACA GTC CA
... ... ... ... ... ..T ... ..
AS, 3361-3339, 3356-3334, 3325-3303, 3712-3690,
3628-3606,
 
MMy31 : CAT GGG TAC CAG CAC ACA AAG G
S, 4186-4207, 4181-4202, 4150-4171, 4534-4555,
4450-4471,
 
MMy31bis : CCT TTG TGT GCT GGT ACC CAT G
AS, 4207-4186, 4202-4181, 4171-4150, 4555-4534,
4471-4450,
 
MMy32 : TGG AAA GGT GAA GGG GCA GT
... ... ... ... ..A ... ..
S, 4992-5011, 4987-5006, 4956-4975, 5340-5359,
5256-5275,
 
MMy32bis : ACT GCC CCT TCA CCT TTC CA
... ... ... ..T ... ... ..
... ... ... ..C ... ... ..
AS, 5011-4992, 5006-4987, 4975-4956, 5359-5340,
5275-5256
2°) séquences communes aux génomes des virus HIV-2 et SIV (les séries de chiffres espacées d'un trait indiquent la position des nucléotides sur les génomes correspondant respectivement aux virus HIV-2 ROD, et SIV-MAC).
   . séquences spécifiques du gène nef2
      (codant pour un facteur négatif de 27 kD) 
MMy12 : AGA GAC TCT TGC GGG CGC GTG
S, 9165-9185, 9139-9159,
 
MMy13 : ATA TAC TTA GAA AAG GAA GAA GG
S, 9542-9564, 9516-9538,
 
MMyl3bis :   CCT TCT TCC TTT TCT AAG TAT AT
AS, 9564-9542, 9538-9516,
 
MMy14 : AGC TGA GAC AGC AGG GAC TTT CCA
AS, 9956-9933, 9893-9870,
   . séquences spécifiques du gène vif2 (codant pour un facteur d'infectiosité de 23 kD) 
MMy20 : TAT GGA GGA GGA AAA GAG ATG GAT AGT
S, 5424-5450, 5340-5366,
 
MMy21 : TAG CAC TTA TTT CCC TTG CTT T
S, 5754-5775, 5670-5691,
 
MMy21bis : AAA GCA AGG GAA ATA AGT GCT A
AS, 5775-5754, 5691-5670,
 
MMy22 : CCC TTG TTC ATC ATG CCA GTA T
AS, 6082-6061, 5995-5974,
   . séquences spécifiques du gène vpx (codant pour une protéine de 12 kD) 
MMy23 : ATG TCA GAT CCC AGG GAG A
S, 5900-5918, 5813-5831,
 
MMy24 : CCT GGA GGG GGA GGA GGA GGA
AS, 6228-6208, 6141-6121,
3°) Séquences communes aux génomes des virus HIV-1 Bru, HIV-1 Mal, et HIV-1 Eli (les séries de chiffres espacées d'un trait indiquent la position des nucléotides sur les génomes correspondant respectivement aux virus HIV-1 Bru, HIV-1 MAl et HIV-1 Eli).
   . séquences spécifiques du gène env (codant pour les protéines d'enveloppe) 
MMy5 : CCA ATT CCC ATA CAT TAT TGT GCC CC
S,6905-6930,6903-6928,6860-6885
 
MMy5bis : GGG GCA CAA TAA TGT ATG GGA ATT GG
AS, 6930-6905, 6928-6903, 6885-6860,
 
MMy6 : AAT GGC AGT CTA GCA GAA GAA GA
S,7055-7077,7053-7075,7010-7032
 
MMy7 : ATC CTC AGG AGG GGA CCC AGA AAT T
S, 7360-7384, 7349-7373,7306-7330
 
MMy7bis : AAT TTC TGG GTC CCC TCC TGA GGA T
AS, 7384-7360,7373-7349,7330-7306
 
MMy8 : GTG CTT CCT GCT GCT CCC AAG AAC CC
AS, 7857-7832,7846-7821,7800-7775
 
MMy8bis : GGG TTC TTG GGA GCA GCA GGA AGC AC
S, 7832-7857, 7821-7846, 7775-7800,
 
MMy9 : ATG GGT GGC AAG TGG TCA AAA AGT AG
... ... ... ..A ... ... ... ... ..
S,8844-8869, 8836-8861, 8787-8812,
 
MMy9bis : CTA CTT TTT GAC CAC TTG CCA CCC AT
AS, 8869-8844, 8861-8836, 8812-8787,
 
MMy78 : TAT TAA CAA GAG ATG GTG G
S, 7629-7647, 7612-7630, 7572-7590,
 
MMy89 : CCA GCA AGA AAA GAA TGA A
S, 8224-8242, 8213-8231, 8167-8185,
 
MMy89bis : TTC ATT CTT TTC TTG CTG G
AS, 8242-8224, 8231-8213, 8185-8167,
   . séquences spécifiques du gène nef1 
MMy10 : AAA AGA AAA GGG GGG ACT GGA
S, 9116-9136, 9117-9137, 9062-9082,
 
MMy10bis : TCC AGT CCC CCC TTT TCT TTT
AS,9136-9116, 9137-9117, 9082-9062,
 
MMy11 : AAA GTC CCC AGC GGA AAG TCC C
AS,9503-9483, 9505-9484, 9449-9428,
   . séquences spécifiques du gène vif 1 
MMy15 : GAT TAT GGA AAA CAG ATG GCA GGT GAT
S, 5073-5099, 5068-5094, 5037-5063,
 
MMy16 : GCA GAC CAA CTA ATT CAT CTG TA
S, 5383-5405, 5378-5400, 5347-5369,
 
MMy16bis : TAC AGA TGA ATT AGT TGG TCT GC
AS, 5405-5383, 5400-5378, 5369-5347,
 
MMy17 : CTT AAG CTC CTC TAA AAG CTC TA
AS, 5675-5653, 5670-5648, 5639-5617,
   . séquences spécifiques du gène vpu

MMy25 : GTA AGT AGT ACA TGT AAT GCA ACC T
S, 6081-6105, 6076-6100, 6045-6069,

MMy26 : AGC AGA AGA CAG TGG CCA TGA GAG
S, 6240-6263, 6238-6261, 6207-6230,

MMy27 : ACT ACA GAT CAT CAA TAT CCC AA
AS, 6343-6321, 6338-6316, 6307-6285,

Ainsi, l'invention vise un oligonucléotide, de 15 à 30 nucléotides, caractérisé en ce que sa séquence consiste en une séquence spécifique du gène *pol*, et susceptible d'hybrider à une température de 60°C ± 1°C avec les génomes des virus HIV-1 Bru, HIV-1 Mal, HIV-1 Eli, HIV-2 Rod et SIV Mac ; l'invention a également pour objet les séquences (ou amorces) possédant une structure nucléotidique complémentaire de celles de l'invention.

En particulier, l'oligonucléotide spécifique du gène pol est choisi parmi
-5'-TAA AGC CAG GAA TGG ATG GCC CAA-3',
-5'-TAA AGC CAG GAA TGG ATG GAC CAA-3',
-5'-TGG ACT GTC AAT GAC ATA CAGAA-3',
-5'-TGG ACT GTC AAT GAT ATA CAGAA-3',
-5'-CAT GGG TAC CAG CAC ACAAAG G-3',
-5'-TGG AAA GGT GAA GGG GCA GT-3',
-5'-TGG AAA GGT GAAGGA GCA GAT-3',
-3'-TTG GGC CAT CCA TTC CTG GCT TTA-5',
-3'-TTG GTC CAT CCA TTC CTG GCT TTA-5',
-3'-TTC TGT ATG TCA TTG ACA GTC CA-5',
-3'-TTC TGT ATG TCA TTG ACT GTC CA-5',
-3'-CCT TTG TGT GCT GGT ACC CAT G-5',
-3'-ACT GCC CCT TCA CCT TTC CA-5',
-3'-ACT GCC CCT TCT CCT TTC CA-5', et
-3'-ACT GCC CCT TCC CCT TTC CA-5'.

L'invention concerne également les séquences nucléotidiques présentant certaines mutations par rapport aux séquences de l'invention définies ci-dessus sans que les propriétés d'hybridation, telles que définies ci-dessus, de ces séquences soient modifiées. Le pourcentage de nucléotides différents de ceux constituant les séquences décrites ci-dessus, sans pour autant affecter les propriétés d'hybridation des séquences de l'invention, peut atteindre 40 %.

D'une manière générale, dans le cas d'une amorce (primer) sens (S), un plus grand nombre de mutations seront tolérées du côté 5' que du côté 3' de l'amorce, le côté 3' devant s'hybrider parfaitement avec un brin déterminé d'une séquence nucléique pour permettre l'amplification de cette séquence. Dans le cas d'une amorce anti-sens(AS), la tolérance est permise du côté 3'.

L'invention a également pour objet les amorces de l'invention telles que définies ci-dessus et comportant une conservation d'au moins 5 bases de chaque côté, la partie médiane comportant des modifications, sans que les propriétés d'hybridation ci-dessus soient modifiées.

Une des caractéristiques des amorces oligonucléotidiques de l'invention est de donner une bande d'amplification nette, dépourvue généralement de bandes aspécifiques lorsque les indications techniques d'utilisation décrites dans la présente invention sont mises en oeuvre. Ce fait est dû à la longueur des amorces pouvant atteindre 27 bases ce qui accroît la spécificité d'hybridation, ainsi qu'aux conditions d'utilisation drastiques qui permettent d'éliminer les associations parasites. La spécificité pour chaque type de virus est fonction, outre du pourcentage d'homologie avec la matrice de référence, de la longueur des amorces, qui peuvent atteindre, pour un rendement acceptable, jusqu'à 40 bases.

L'invention s'étend également aux amorces de l'invention telles que décrites ci-dessus liées au niveau de leur extrémité 5' à un promoteur pour la mise en oeuvre d'une méthode d'amplification génomique par synthèse de multiple copies d'ADN ou d'ARN telle que décrite dans la demande de brevet européenne EP 310229.

L'invention a notamment pour objet l'utilisation des amorces de l'invention décrites ci-dessus pour la mise en oeuvre d'un procédé d'amplification génique de séquences nucléiques de virus du type HIV-1 et/ou HIV-2, et/ou SIV, ce procédé étant applicable au diagnostic in vitro de l'infection potentielle d'un individu par un virus du type HIV-1 et/ou HIV-2 ou d'un animal par au moins l'un des trois virus (HIV-1, HIV-2, SIV).

Cette méthode de diagnostic in vitro est réalisée à partir d'un échantillon biologique (par exemple un fluide biologique tel que le sérum, les lymphocytes du sang circulant) obtenu à partir d'un patient à l'étude, et comprend principalement les étapes suivantes :
- une étape d'extraction de l'acide nucléique à détecter appartenant au génome du virus du type HIV-1 et/ou HIV-2 et/ou SIV éventuellement présent dans l'échantillon biologique sus-mentionné, et,le cas échéant, une étape de traitement à l'aide d'une transcriptase inverse dudit acide nucléique si ce dernier est sous forme d'ARN afin d'obtenir un acide nucléique double brin (cette dernière étape étant encore désignée ci-dessous par étape de rétro-transcription de l'ARN viral),
- un cycle comprenant les étapes suivantes :
   - dénaturation de l'acide nucléique double brin à détecter , ce qui conduit à la formation d'un acide nucléique simple brin,
   - hybridation de chacun des brins d'acide nucléique, obtenus lors de l'étape de dénaturation précédente, avec au moins une amorce selon l'invention, par mise en contact des brins sus-mentionnés avec au moins un couple d'amorces selon l'invention dans les conditions d'hybridation définies ci-dessous,
   - formation à partir des amorces des ADN complémentaires aux brins sur lesquels elles sont hybridées en présence d'un agent de polymérisation (ADN polymérase) et de quatre nucléosides triphosphate (dNTP) différents, ce qui conduit à la formation d'un plus grand nombre d'acides nucléiques double brin à détecter qu'à l'étape de dénaturation précédente,
      ce cycle étant répété un nombre de fois déterminé pour obtenir ladite séquence nucléique à détecter éventuellement présente dans l'échantillon biologique dans une proportion suffisante pour permettre sa détection,
- une étape de détection de la présence éventuelle de l'acide nucléique appartenant au génome du virus du type HIV-1 et/ou HIV-2 et/ou SIV dans l'échantillon biologique.

L'étape d'hybridation décrite ci-dessus est avantageusement réalisée à 60°C pendant 1 minute 30 secondes dans le tampon "10 X buffer" dont la composition (en concentration finale d'utilisation) est indiquée ci-dessous.

La méthode de diagnostic in vitro peut être réalisée soit à partir de l'ARN viral, soit à partir de l'ADN complémentaire, épisomial ou intégré.

En effet, les génomes des virus HIV et SIV se présentent sous forme d'ARN ou d'ADN en fonction de la localisation du virus dans l'organisme.

Lorsque le virus est situé à l'intérieur des cellules de l'organisme, notamment à l'intérieur des cellules sanguines, son ARN est recopié en ADN par une transcriptase inverse. En revanche, le génome des virus du type HIV en milieu extracellulaire, notamment dans le sang, demeure sous forme d'ARN.

L'étape d'extraction de l'ADN viral contenu dans les cellules de l'échantillon biologique préconisée par les inventeurs - outre la méthode classique au phénol chloroforme - présente les étapes suivantes :
- suspension du culot cellulaire dans 0,5 ml d'eau pyrolisée dans un Potter gros piston,
- broyage des cellules dit par "aller et retour",
- adjonction Triton X100 pour 1 concentration finale de 0, 1 %,
- dénaturation à la chaleur durant 15 à 25 minutes à 100°C,
- centrifugation courte pour n'éliminer que les débris cellulaires,
- précipitation de l'ADN durant la nuit à -20°C par adjonction de 2,5 volumes d'éthanol absolu et de 10 % du volume final d'acétate de sodium 3 Molaires. L'ADN est ensuite récupéré puis resuspendu dans l'eau pyrolysée après avoir été lavé 2 fois par de l'éthanol à 70°. Il est à noter que cette méthode permet la précipitation conjointe des ADN et des ARN, ce qui autorise la détection du message génomique des virus de types HIV ou SIV par utilisation de la méthode dite "PCR directe ADN" ou par celle dite de "PCR-ARN".

L'étape d'extraction de l'ARN viral est généralement effectuée de manière classique connue de l'homme de l'Art.

Après extraction de l'ARN, une étape supplémentaire de transformation de l'ARN, monobrin en ADN double brin est nécessaire à effectuer lorsque le diagnostic in vitro est réalisé à partir d'échantillons biologiques contenant les virus du type HIV-1 et/ou HIV-2 et/ou SIV dont les génomes sont sous forme d'ARN.

Cette transformation de l'ARN en ADN est réalisée par traitement de l'ARN obtenu après extraction de l'échantillon biologique, notamment du sérum, dans un milieu approprié à l'aide d'une transcriptase inverse.

Est plus particulièrement décrite une méthode de diagnostic in vitro telle que définie ci-dessus, dans laquelle l'étape de rétro-transcription de l'ARN viral est réalisée de la manière suivante :
- 10 µg d'ARN extrait resuspendu dans de l'eau est mis en présence du couple d'amorces à la concentration de 40 µM chacun, dans un volume final de 40 µl. L'ensemble est dénaturé à 100°C durant 10 minutes puis plongé dans de l'eau glacée,
- l' on rajoute 10 µl du mélange suivant : 5 µl du tampon "10 X buffer" décrit ci-dessous + 1 unité de reverse-transcriptase (d'AMV (Avian Myeloblastosis Virus) ou de MuMLV (Moloney Leukemia Virus)) + 1 unité de Taq-polymérase + 1 µl du mélange des 4 dNTP à 25 mM chacun + de l'eau Q.S.P. 10 µl. Le volume final est donc de 50 µl.

Cette réaction s'effectue en deux étapes :
- a) 1ère étape : fabrication de l'ADNc par action de la réverse transcriptase à 42°C durant 13 minutes,
- b) 2ème étape : amplification génique classique: on chauffe à 95°C durant 3 minutes pour détruire la réverse-transcriptase et permettre l'étape de déshybridation/hybridation, puis on démarre le cycle décrit précédemment pour l'amplification génique.

Est particulièrement décrite une méthode de diagnostic in vitro telle que décrite çi-dessus, dans laquelle l'étape de dénaturation est réalisée en présence du (ou des) couple(s) d'amorces (ou de primers) décrits. En effet, comme il a été précisé ci-dessus, une des caractéristiques des oligonucléotides (ou primers) décrits est de donner une bande d'amplification nette, dépourvue généralement de bandes aspécifiques, lorsqu'ils sont utilisés dans les conditions qui suivent :
- hybridation : les primers (1µl d'une solution à 40 µmolaire (40 µM) de chaque primer) sont mis en présence de l'ADN-matrice (100 à 300 ng) pour la première étape de dénaturation-réassociation ; on chauffe, durant 10 minutes à 100°C puis on plonge les tubes contenant ce mélange d'ADN-matrice et de primers dans de l'eau contenant de la glace afin d'augmenter le taux de réassociation ADN-matrice/primers. Les primers doivent être utilisés à une concentration finale dans l'étape d'amplification qui suit de 0,8 µM chaque.
- amplification : on ajoute au milieu précédent les 4 A dNTP chacun étant utilisé à 0,5 µmolaire en solution finale (50 µl), et une unité de Taq-polymérase pour un milieu réactionnel de 50 µl ; cette étape est réalisée dans un tampon d'amplification de la présente invention, généralement désigné sous le nom de "10 X buffer" dont la composition (lorsqu'il est dilué au 1/10°) est la suivante : Tris-HCl, pH 8,9 : 50 mM ; (NH₄)₂ SO₄ : 15 mM ; MgCl2 : 5 mM ; β-mercapto-éthanol : 10 mM ; gélatine : 0,25 mg/ml. On additionne 5 µl de ce tampon et de l'eau q.s.p. 50 µl au milieu précédent.

Les cycles d'amplification sont réalisés de la manière suivante : 30 à 40 cycles composés de :
. 94°C durant 10 secondes (dénaturation),
. 60°C durant 1 minute 30 (hybridation),
. 78°C durant 1 minute 30 (élongation).

Le tout sera suivi par un cycle unique à 78°C durant 15 minutes.

La précision des températures indiquées à ± 0,3°C près, ainsi que leur stabilité durant les différents cycles, représentent des conditions essentielles pour l'obtention des rendements maximum ainsi que l'absence de bandes aspécifiques.

La concentration optimale d'ADN est de 100 à 300 ng pour de l'ADN génomique extrait de cellules (de patients ou en culture, de mammifères ou autres).

Il va de soi que les conditions qui précèdent représentent des conditions optimales pour un milieu réactionnel final de 50 µl, et que ces conditions peuvent être modifiées en fonction du volume final du milieu réactionnel.

L'utilisation de plusieurs couples d'amorces différents (ou coktails de couples) permet soit la détection croisée de plusieurs types de virus du type HIV et/ou SIV, soit la détection simultanée de plusieurs gènes d'un même virus du type HIV et/ou SIV.

A titre d'exemple de couples d'amorces, on peut citer les couples d'amorces suivants :
- MMy1-MMy4, MMy2-MMy4, MMy1-MMy3, MMy18-MMy19, MMy4bis-MMy28bis, MMy28-MMy29bis, MMy29-MMy30bis, MMy31-MMy32bis, notamment pour le diagnostic in vitro de l'infection d'un individu par HIV-1 et/ou HIV-2
- MMy5-MMy8, MMy6-MMy8, MMy7-MMy8, MMy5-MMy7bis, MMy6-MMy7bis, MMy9-MMy11, HMy10-My11, MMy9-HMy10bis, MMy26-MMy5bis, MMy8bis-MMy9bis, MMy8bis-HMy89, MMy89bis-MMy9bis, MMy15-MMy17, MMy15-MMy16bis, MMy16-MMy17, MMy25-MMy27, MMy26-MMy27, notamment pour le diagnostic in vitro de l'infection d'un individu par HIV-1,
- MMy20-MMy22, MMy20-MMy21bis, MMy21-MMy22, MMy23-MMy24, MMy12-MMy14, MMy12-MMy13bis, pour le diagnostic in vitro de l'infection d'un individu par HIV-2.

L'agent de polymérisation utilisé dans l'étape d'élongation du cycle est une ADN polymérase thermostable, notamment la Taq polymérase, l'amplifiose de la firme Appligène ou toute ADN-polymérase thermostable pouvant être commercialisée.

D'une manière générale, le cycle de la méthode de diagnostic in vitro de l'invention est répété entre 30 et 40 fois.

La méthode de diagnostic in vitro de l'invention permet également, en fonction des couples d'amorces nucléotidiques utilisés, de détecter sélectivement les gènes des virus du type HIV et/ou SIV présents dans l'échantillon biologique.

Les couples d'amorces utilisables, à titre d'exemples, pour une methode de diagnostic gène par gène, sont les suivants :
- MMy1-MMy4, MMy2-MMy4, MMy1-MMy3, MMy4bis-MMy28bis pour le gène gag,
- MMy18-MMy19 pour le gène vpr,
- MMy5-MMy8, MMy6-MMy8, MMy7-MMy8, MMy5-MMy7bis, MMy6-MMy7bis, MMy26-MMy5bis, MMy8bis-MMy9bis, MMy8bis-MMy89, MMy89bis-MMy9bis pour le gène env,
- MMy9-MMy11, MMy9-MMy10bis, MMy10-MMy11 pour le gène nef1,
- MMy15-MMy17, MMy15-MMy16bis, MMy16-MMy17, pour le gène vif1,
- MMy20-MMy22, MMy20-MMy21bis, MMy21-MMy22 pour vif 2,
- MMy23-MMy24 pour vpx,
- MMy12-MMy14, MMy12-MMy13bis, MMy13-MMy14 pour nef2,
- MMy25-MMy27, MMY26-MMy27 pour le gène vpu,
- MMy28-MMy29bis, MMy29-MMy30bis, MMy30-MMy31bis, MMy31-MMy32bis pour le gène pol.

Dans le cadre de l'invention, des couples d'amorces utilisés pour la mise en oeuvre d'une amplification du gène *pol* des virus de type HIV-1, HIV-2 et SIV consistent en :
i) MMy29-MMy30bis
   5'-TAA AGC CAG GAA TGG ATG GCC CAA-3'
   5'-TAA AGC CAG GAA TGG ATG GAC CAA-3'
   3'-TTC TGT ATG TCA TTG ACA GTC CA-5'
   3'-TTC TGT ATG TCA TTG TACT GTC CA-5'
ii) Mmy30-Mmy31bis
   5'-GG ACT GTC AAT GAC ATA CAGAA-3'
   5'-TGG ACT GTC AAT GAT ATA CAGAA-3'
   3'-CCT TTG TGT GCT GGT ACC CAT G-5'
iii) Mmy31-Mmy32bis:
   5'-CAT GGG TAC CAG CAC ACAAAG G-3'
   3'-ACT GCC CCT TCA CCT TTC CA-5'
   3'-ACT GCC CCT TCT CCT TTC CA-5'
   3'-ACT GCC CCT TCC CCT TTC CA-5'

Toutefois, les combinaisons entre primers "S" et "AS" décrites ci-dessus ne sont par limitatives et peuvent être variées selon le désir de l'utilisateur.

Les tailles des fragments nucléotidiques synthétisés à l'aide des couples d'amorces susmentionnés à titre d'exemples, sont indiquées sur les tableaux I à XI suivants :
(les chiffres indiqués dans les tableaux ci-dessous représentent le nombre de nucléotides des fragments synthétisés, et les "tirets" indiquent que les couples d'amorces testés ne permettent pas de caractériser les souches virales correspondantes).

**Tableau I**

| | gag | | gag | |
|---|---|---|---|---|
| | MMy1-MMy3 | MMy1-MMy4 | MMy2-MMy4 | MMy4bis-MMy28bis |
| HIV1-BRU | 265 | 750 | 532 | 671 |
| HIV1-MAL | 282 | 785 | 556 | 671 |
| HIV1-ELI | 265 | 750 | 538 | 674 |
| HIV2-ROD | 354 | 845 | 544 | 663 |
| SIV | 343 | 844 | 544 | 668 |

**Tableau II**

| | env | | env | |
|---|---|---|---|---|
| | MMy5-MMy7bis | MMy5-MMy8 | MMy6-MMy7bis | MMy6-MMy8 |
| HIV1-BRU | 480 | 953 | 330 | 803 |
| HIV1-MAL | 471 | 944 | 321 | 794 |
| HIV1-ELI | 471 | 941 | 321 | 791 |
| HIV2-ROD | - | - | - | - |
| SIV | - | - | - | - |

**Tableau III**

| | env | | env |
|---|---|---|---|
| | MMy7-MMy8 | MMy26-MMy5bis | MMy8bis-MMy9bis |
| HIV1-BRU | 498 | 691 | 1038 |
| HIV1-MAL | 498 | 691 | 1041 |
| HIV1-ELI | 495 | 679 | 1038 |
| HIV2-ROD | - | - | - |
| SIV | - | - | - |

**Tableau IV**

| | env | env |
|---|---|---|
| | MMy8bis-MMy89 | MMy89bis-MMy9bis |
| HIV1-BRU | 411 | 646 |
| HIV1-MAL | 411 | 649 |
| HIV1-ELI | 411 | 646 |
| HIV2-ROD | - | - |
| SIV | - | - |

**Tableau V**

| | nef1 | nef1 | |
|---|---|---|---|
| | MMy9-MMy10bis | MMy9-MMy11 | MMy10-MMy11 |
| HIV1-BRU | 293 | 660 | 388 |
| HIV1-MAL | 302 | 660 | 388 |
| HIV1-ELI | 296 | 663 | 388 |
| HIV2-ROD | - | - | - |
| SIV | - | - | :- |

**Tableau VI**

| | nef2 | nef2 | |
|---|---|---|---|
| | MMy12-MMy13bis | MMy12-MMy14 | MMy13-MMy14 |
| HIV1-BRU | - | - | |
| HIV1-MAL | - | - | - |
| HIV1-ELI | - | - | - |
| HIV2-ROD | 400 | 792 | 415 |
| SIV | 400 | 755 | 378 |

**Tableau VII**

| | vif1 | vif1 | |
|---|---|---|---|
| | MMy15-MMy16bis | MMy15-MMy17 | MMy16-MMy17 |
| HIV1-BRU | 333 | 603 | 293 |
| HIV1-MAL | 333 | 603 | 293 |
| HIV1-ELI | 333 | 603 | 293 |
| HIV2-ROD | - | - | - |
| SIV | - | - | - |

**Tableau VIII**

| | vpr | vif2 | |
|---|---|---|---|
| | MMy18-MMy19 | MMy20-MMy21bis | MMy20-MMy22 |
| HIV1-BRU | 281 | - | - |
| HIV1-MAL | 281 | - | - |
| HIV1-ELI | 281 | - | - |
| HIV2-ROD | 319 | 352 | 659 |
| SIV | 308 | 352 | 656 |

**Tableau IX**

| | vif2 | vpx |
|---|---|---|
| | MMy21-MMy22 | MMy23-MMy24 |
| HIV1-BRU | - | - |
| HIV1-MAL | - | - |
| HIV1-ELI | - | - |
| HIV2-ROD | 329 | 329 |
| SIV | 326 | 329 |

**Tableau X**

| | vpu | pol | |
|---|---|---|---|
| | MMy25-MMy27 | MMy26-MMy27 | MMy28-MMy29bis |
| HIV1-BRU | 263 | 104 | 623 |
| HIV1-MAL | 263 | 101 | 584 |
| HIV1-ELI | 263 | 101 | 584 |
| HIV2-ROD | - | - | 666 |
| SIV | - | - | 712 |

**Tableau XI**

| | pol | pol | |
|---|---|---|---|
| | MMy29-MMy30bis | MMy30-MMy31bis | MMy31-MMy32bis |
| HIV1-BRU | 742 | 869 | 826 |
| HIV1-MAL | 742 | 869 | 826 |
| HIV1-ELI | 742 | 869 | 826 |
| HIV2-ROD | 742 | 866 | 826 |
| SIV | 742 | 866 | 826 |

Il est à noter que grâce à leur disposition sur le génome, les primers servant à l'amplification peuvent être combinés de telle façon qu'ils peuvent être utilisés comme sonde, soit après marquage au ³²p par kination, soit pour l'utilisation dans la technique des sondes froides pour vérifier la spécificité de la bande d'amplification observée lors d'une analyse par "Southern transfert". En plus de la combinaison classique des amorces pour qu'un troisième oligonucléotide puisse servir de sonde interne spécifique, il est à noter le cas particulier des gènes vif1/vpr et vif2/vpx dû au chevauchement de ces gènes, ce qui permet une détection croisée. En outre, lors d'une analyse par séquençage de l'ADN amplifié, ces oligonucléotides peuvent être utilisés comme amorce spécifique pour la DNA-polymérase permettant un double séquençage dans chaque sens, donc une double lecture des séquences, levant ainsi des ambiguïtés éventuelles d'interprétation.

L'invention a également pour objet les amorces de l'invention telles que définies ci-dessus, marquées, notamment de manière radioactive ou enzymatique, ainsi que leur utilisation en tant que sondes nucléotidiques, notamment dans le cadre de la méthode de diagnostic in vitro telle que décrite ci-dessus.

L'invention a également pour objet des oligonucléotides tels que ceux de l'invention décrits ci-dessus et comportant des sucres en conformation a. De tels oligonucléotides présentent la caractéristique d'inverser le sens de la double hélice formée avec la matrice (brin du génome du virus), cette double hélice passant ainsi de l'état "S" à l'état "AS".

L'invention concerne aussi les oligonucléotides tels que ceux de l'invention décrits ci-dessus dont certains nucléotides sont méthylés et/ou comportent un ou plusieurs atomes de soufre notamment sur les adénines. De tels oligonucléotides présentent la caractéristique d'augmenter la stabilité de la double hélice, et par conséquent de mieux s'hybrider avec le brin d'ADN à amplifier.

L'invention concerne également les oligonucléotides tels que ceux de l'invention décrits ci-dessus et se présentant sous la forme dite "de bases modifiées" comportant des nucléotides sur lesquels sont greffés de façon covalente des agents chromophores (molécules aromatiques planes telles que l'acridine orange), notamment selon la méthode décrite dans l'article de C. Hélène paru dans "la Vie des Sciences", compte-rendus, série générale, tome 4, n°1, p. 17-37. De tels oligonucléotides présentent la caractéristique d'être facilement détectables, notamment par fluorescence.

Les oligonucléotides de l'invention sont également utilisables pour la mise en oeuvre d'une méthode de diagnostic in vitro de l'infection de singes (macaque, singe de mangabeys ou singe vert) par le virus du type SIV, cette méthode reprenant les principales caractéristiques de celle décrite ci-dessus.

L'invention a également pour objet des kits de diagnostic pour la mise en oeuvre des méthodes de diagnostic in vitro sus-mentionnées. A titre d'exemple, un kit de diagnostic de la présente invention comprend :
- au moins un couple d'amorces oligonucléotidiques selon l'invention, chaque couple comprenant une amorce s'hybridant à l'un des brins de la séquence d'acide nucléique à détecter, et une amorce s'hybridant avec le brin complémentaire de ce dernier dans les conditions définies ci-dessus,
- des réactifs appropriés à la mise en oeuvre du cycle d'opérations d'amplification, notamment de l'ADN polymérase, et quatre nucléotides triphosphate différents, et le milieu réactionnel dénommé "10 X buffer" décrit ci-dessus.
- une (ou plusieurs) sonde pouvant être marquée, notamment par radioactivité ou par la technique des sondes froides, capable de s'hybrider spécifiquement avec la (ou les) séquence(s) d'acides nucléiques amplifiée(s) à détecter.

La demande décrit également l'utilisation des amorces indiquées ci-dessus pour la mise en oeuvre d'un procédé de synthèse de protéines codées par les séquences nucléotidiques amplifiées à l'aide de ces amorces.

A titre illustratif, ce procédé de synthèse de protéines comprend l'amplification de séquences nucléotidiques des génomes des virus du type HIV ou SIV (codant pour une protéine déterminée et ayant subi, le cas échant, certaines modifications de leurs nucléotides) par mise en contact desdites séquences avec au moins un couple d'amorces selon l'invention dans les conditions décrites ci-dessus, suivie de la traduction de ces séquences ainsi amplifiées en protéines ; cette dernière étape est réalisée notamment par transformation de cellules hôtes appropiées à l'aide de vecteurs contenant lesdites séquences amplifiées, et récupération des protéines produites dans ces cellules hôtes.

La demande décrit également les polypeptides issus de la traduction des séquences (ou amorces) nucléotidiques décrites ci-dessus.

La demande divulgue aussi l'utilisation des amorces oligonucléotidiques anti-sens en tant qu'agents antiviraux en général, notamment dans la lutte contre le SIDA, ainsi que des compositions pharmaceutiques contenant ces amorces anti-sens en association avec un véhicule pharmaceutiquement acceptable.

La demande décrit également les compositions immunogènes comprenant un ou plusieurs produits de traduction des séquences nucléotidiques, et/ou un ou plusieurs produits de traduction des séquences nucléotidiques amplifiées selon les procédés décrits ci-dessus à partir des amorces définies ci-dessus, ces produits de traduction étant associés à un véhicule pharmaceutiquement acceptable.

La demande décrit les anticorps dirigés contre l'un ou plusieurs des produits de traduction décrits ci-dessus (ou en d'autres termes, susceptibles de former une réaction immunologique avec un ou plusieurs produits de traduction des séquences nucléotidiques ci-dessus, ou encore un ou plusieurs produits de traduction des séquences nucléotidiques amplifiées à partir des amorces définies ci-dessus) et leur utilisation pour la mise en oeuvre de méthodes de diagnostic in vitro de l'infection d'un individu par un virus du type HIV-1 et/ou HIV-2, ou d'un animal par au moins l'un des trois virus (HIV-1, HIV-2, SIV) selon les procédés connus de l'homme de l'Art.

A titre illustratif, une telle méthode de diagnostic in vitro comprend la mise en contact d'un échantillon biologique (notamment de sérum) prélevé chez un patient à l'étude, avec des anticorps, et la détection à l'aide de tout procédé approprié (notamment à l'aide d'anti-immunoglobulines marquées) des complexes immunologiques formés entre les antigènes des virus du type HIV ou SIV éventuellement présents dans l'échantillon biologique et lesdits anticorps.

La demande décrit également des kits de diagnostic in vitro comprenant des anticorps tels que définis ci-dessus et, le cas échéant, des réactifs appropriés à la mise en évidence de la réaction immunologique formée entre lesdits anticorps et les antigènes des virus HIV ou SIV.

La demande rapporte également un procédé de préparation des polypeptides mentionnés ci-dessus, notamment ceux correspondant selon le code génétique universel aux séquences (ou amorces) nucléotidiques décrites ci-dessus, ce procédé étant caractérisé en ce que, partant de préférence de l'aminoacide C-terminal, l'on condense successivement deux à deux les aminoacyles successifs dans l'ordre requis, ou des aminoacyles et des fragments préalablement formés et contenant déjà plusieurs résidus aminoacyles dans l'ordre approprié, ou encore plusieurs fragments préalablement ainsi préparés, étant entendu que l'on aura eu soin de protéger au préalable toutes les fonctions réactives portées par ces aminoacyles ou fragment à l'exception des fonctions amine de l'un et carboxyle de l'autre ou vice-versa, qui doivent normalement intervenir dans la formation des liaisons peptidiques, notamment après activation de la fonction carboxyle, selon les méthodes connues dans la synthèse des peptides et ainsi de suite, de proche en proche, jusqu'à l'acide aminé N-terminal.

Par exemple, on aura recours à la technique de synthèse peptidique en solution homogène décrite par Houbenweyl dans "Methode der Organischen Chemie" (Méthode de la Chimie Organique) édité par E. Wunsch, vol. 15-I et II, THIEME, STUTTGART, 1974, ou à celle de synthèse peptidique en phase solide décrite par R.D. Merrifield dans "Solid Phase Peptide Synthesis" (J. AM. CHEM. SOC., 45, 2149-2154).

La demande divulgue également un procédé de préparation des séquences (ou amorces) nucléotidiques décrites ci-dessus, ce procédé comprenant les étapes suivantes :
- incubation de l'ADN génomique, isolé à partir d'un des virus du type HIV ou SIV sus-mentionnés, avec de l'ADNase I, puis addition d'EDTA et purification par extraction au mélange phenol/chloroforme/alcool isoamylique (25/24/ 1) puis par l'éther,
- traitement de l'ADN ainsi extrait par de l'Eco R1 méthylase en présence de DTT, et purification par extraction telle que décrite ci-dessus,
- incubation de l'ADN ainsi purifié avec les 4 désoxynucléotides triphosphates dATP, dCTP, dGTP, et dTTP en présence de T4 ADN polymérase et d'ADN ligase de E. coli, puis purification selon la méthode décrite ci-dessus,
- le clonage des acides nucléiques ainsi obtenus dans un vecteur approprié et la récupération de l'acide nucléique recherché à l'aide d'une sonde appropriée.

Un procédé de préparation particulièrement avantageux des séquences nucléotidiques comprend les étapes suivantes :
- la synthèse d'ADN en utilisant la méthode automatisée des β-cyanethyl phosphoramidite décrite dans Bioorganic Chemistry 4; 274-325 (1986),
- le clonage des acides nucléiques ainsi obtenus dans un vecteur approprié et la récupération de l'acide nucléique par hybridation avec une sonde appropriée.

Un autre procédé de préparation des séquences nucléotidiques comprend les étapes suivantes :
- l'assemblage d'oligonucléotides synthétisés chimiquement, pourvus à leurs extrémités de sites de restriction différents, dont les séquences sont compatibles avec l'enchaînement en acides aminés du polypeptide naturel selon le principe décrit dans Proc. Natl. Acad. Sci. USA, 80; 7461-7465, (1983),
- le clonage des acides nucléiques ainsi obtenus dans un vecteur approprié et la récupération de l'acide nucléique recherché par hybridation avec une sonde appropriée.

## Revendications

1. Oligonucléotide, de 15 à 30 nucléotides, **caractérisé en ce que** sa séquence consiste en :
i) une séquence spécifique du gène *pol*, et susceptible d'hybrider à une température de 60°C ± 1°C avec les génomes des virus HIV-1 Bru, HIV-1 Mal, HIV-1 Eli, HIV-2 Rod et SIV Mac, ou
ii) une séquence complémentaire d'une séquence telle que définie en i.

2. Oligonucléotide, de 15 à 30 nucléotides, **caractérisé en ce que** sa séquence nucléotidique est modifiée par rapport à la séquence de l'oligonucléotide selon la revendication 1 et garde les propriétés d'hybridation avec les génomes des virus HIV-1 Bru, HIV-1 Mal, HIV-1 Eli, HIV-2 Rod et SIV Mac à une température de 60°C ± 1 °C

3. Oligonucléotide selon la revendication 2, dont les 5 bases de chaque extrémité sont conservées par rapport à celles d'une amorce oligonucléotidique telle que décrite à la revendication 1, et qui comporte dans sa partie médiane des modifications par rapport à la séquence d'une amorce oligonucléotidique selon la revendication 1.

4. Oligonucléotide spécifique du gène *pol* selon la revendication 1 choisi parmi :
- 5'-TAA AGC CAG GAA TGG ATG GCC CAA-3',
- 5'-TAA AGC CAG GAA TGG ATG GAC CAA-3',
- 5'-TGG ACT GTC AAT GAC ATA CAGAA-3',
- 5'-TGG ACT GTC AAT GAT ATA CAGAA-3',
- 5'-CAT GGG TAC CAG CAC ACAAAG G-3',
- 5'-TGG AAA GGT GAA GGG GCA GT-3',
- 5'-TGG AAA GGT GAAGGA GCA GT-3',
- 3'-TTG GGC CAT CCA TTC CTG GCT TTA-5',
- 3'-TTG GTC CAT CCA TTC CTG GCT TTA-5',
- 3'-TTC TGT ATG TCA TTG ACA GTC CA-5',
- 3'-TTC TGT ATG TCA TTG ACT GTC CA-5',
- 3'-CCT TTG TGT GCT GGT ACC CAT G-5',
- 3'-ACT GCC CCT TCA CCT TTC CA-5',
- 3'-ACT GCC CCT TCT CCT TTC CA-5', et
- 3'-ACT GCC CCT TCC CCT TTC CA-5'.

5. Couple d'amorces oligonucléotidiques, pour la mise en oeuvre d'une amplification génique du gène *pol* de virus de type HIV-1, HIV-2 et SIV, les amorces consistant chacune en :
i) une séquence spécifique du gène *pol*, de 15 à 30 nucléotides, et susceptible d'hybrider à une température de 60°C ± 1°C avec les génomes des virus HIV-1 Bru, HIV-1 Mal, HIV-1 Eli, HIV-2 Rod et SIV Mac
ii) une séquence complémentaire d'une séquence telle que définie en i).

6. Couple d'amorces **caractérisé en ce que** la séquence d'au moins une amorce oligonucléotidique d'un couple d'amorces selon la revendication 5 est modifiée par rapport à la séquence du gène *pol* des virus HIV-1 Bru, HIV-1 Mal, HIV-1 Eli, HIV-2 Rod et SIV Mac, et garde les propriétés d'hybridation avec les génomes des virus HIV-1 Bru, HIV-1 Mal, HIV-1 Eli, HIV-2 Rod et SIV Mac à une température de 60°C ± 1°C.

7. Couple d'amorces selon la revendication 5, pour la mise en oeuvre d'une amplification génique du gène *pol* de virus de type HIV-1, HIV-2 et SIV, les amorces consistant en :
i. au moins un mélange choisi dans le groupe des mélanges de séquences sens suivant :
Mmy29: Mélange constitué des séquences
5'-TAA AGC CAG GAA TGG ATG GCC CAA-3'
5'-TAA AGC CAG GAA TGG ATG GAC CAA-3'
Mmy30: Mélange constitué des séquences
5'-TGG ACT GTC AAT GAC ATA CAGAA-3'
5'-TGG ACT GTC AAT GAT ATA CAGAA-3'
Mmy31: Mélange constitué de la séquence 5'-CAT GGG TAC CAG CAC ACAAAG G-3'
Mmy32: Mélange constitué des séquences
5'-TGG AAA GGT GAA GGG GCA GT-3'
5'-TGG AAA GGT GAAGGA GCA GT-3', et
ii) au moins un mélange choisi dans le groupe des mélanges de séquences antisens suivant :
Mmy29bis: Mélange constitué des séquences
3'-TTG GGC CAT CCA TTC CTG GCT TTA-5'
3'-TTG GTC CAT CCA TTC CTG GCT TTA-5'
Mmy30bis: Mélange constitué des séquences
3'-TTC TGT ATG TCA TTG ACA GTC CA-5'
3'-TTC TGT ATG TCA TTG ACT GTC CA-5'
Mmy31 bis: Mélange constitué de la séquence 3'-CCT TTG TGT GCT GGT ACC CAT G-5'
Mmy32bis: Mélange constitué des séquences
3'-ACT GCC CCT TCA CCT TTC CA-5'
3'-ACT GCC CCT TCT CCT TTC CA-5'
3'-ACT GCC CCT TCC CCT TTC CA-5'.

8. Couple d'amorces selon la revendication 7, pour la mise en oeuvre d'une amplification génique du gène pol de virus de type HIV-1, HIV-2 et SIV, consistant en :
i) Mmy29-Mmy30bis
5'-TAA AGC CAG GAA TGG ATG GCC CAA-3'
5'-TAA AGC CAG GAA TGG ATG GAC CAA-3'
3'-TTC TGT ATG TCA TTG ACA GTC CA-5'
3'-TTC TGT ATG TCA TTG ACT GTC CA-5'
ii) Mmy30-Mmy31 bis
5'-TGG ACT GTC AAT GAC ATA CAGAA-3'
5'-TGG ACT GTC AAT GAT ATA CAGAA-3'
3'-CCT TTG TGT GCT GGT ACC CAT G-5'
iii) Mmy31-Mmy32bis:
5'-CAT GGG TAC CAG CAC ACAAAG G-3'
3'-ACT GCC CCT TCA CCT TTC CA-5'
3'-ACT GCC CCT TCT CCT TTC CA-5'
3'-ACT GCC CCT TCC CCT TTC CA-5'

9. Oligonucléotide ou couple d'amorces oligonucléotidiques selon l'une quelconque des revendications 1 à 8 susceptible d'hybrider avec les génomes des virus HIV-1 Bru, HIV-1 Mal, HIV-1 Eli, HIV-2 Rod et SIV Mac dans un tampon 10X buffer de composition Tris-HCl, pH 8,9 : 50 mM ; (NH₄)₂ SO₄ : 15 mM ; MgCl₂ : 5mM ; β-mercapto-éthanol : 10 mM ; gélatine : 0,25 mg/ml.

10. Procédé d'amplification génique de séquences nucléiques du gène *pol* de virus du type HIV-1 et/ou HIV-2, et/ou SIV, réalisé à partir d'un échantillon biologique, ce procédé comprenant principalement les étapes suivantes:
a) une étape d'extraction de l'acide nucléique à détecter appartenant au génome du virus du type HIV-1, HIV-2, ou SIV éventuellement présent dans l'échantillon biologique sus-mentionné, et,le cas échéant, une étape de traitement à l'aide d'une transcriptase inverse dudit acide nucléique si ce dernier est sous forme d'ARN,
b) un cycle comprenant les étapes suivantes :
. dénaturation de l'acide nucléique double brin à détecter, ce qui conduit à la formation d'un acide nucléique simple brin,
. hybridation de chacun des brins d'acide nucléique, obtenus lors de l'étape de dénaturation précédente, avec au moins une amorce oligonucléotidique ou un couple d'amorces oligonucléotidiques selon l'une des revendications 1 à 9, par mise en contact des brins susmentionnés avec au moins un couple d'amorces oligonucléotidiques susmentionnées,
. formation à partir des amorces des ADN complémentaires aux brins sur lesquels elles sont hybridées en présence d'une ADN polymérase et de quatre nucléosides triphosphate (dNTP) différents, ce qui conduit à la formation d'un plus grand nombre d'acides nucléiques double brin à détecter qu'à l'étape de dénaturation précédente,
ce cycle étant répété un nombre de fois déterminé pour obtenir ladite séquence nucléique à détecter éventuellement présente dans l'échantillon biologique dans une proportion suffisante pour permettre sa détection,
c) une étape de détection de la présence éventuelle de l'acide nucléique appartenant au génome du virus du type HIV-1 et/ou HIV-2 et/ou SIV dans l'échantillon biologique.

11. Procédé selon la revendication 10, **caractérisé en ce que** l'étape de dénaturation est réalisée en présence du (ou des) couple(s) d'amorces oligonucléotidiques selon l'une des revendications 6 à 9.

12. Procédé selon la revendication 11, **caractérisé en ce qu'**il est réalisé dans les conditions suivantes :
a) hybridation : les amorces oligonucléotidiques (1 µl d'une solution à 40 µmonaire de chaque amorce) sont mis en présence de l'ADN-matrice (100 à 300 ng) pour la première étape de dénaturation-réassociation ; on chauffe, durant 10 minutes à 100 °C puis on plonge les tubes contenant ce mélange d'ADN-matrice et d'amorces dans de l'eau contenant de la glace. Les amorces doivent être utilisés à une concentration finale de 0,8 µM chaque, dans l'étape d'amplification qui suit,
b) amplification : on ajoute au milieu précédent les 4 dNTP chacun étant utilisé à 0,5 µmolaire en solution finale (50 µl), et une unité de Taq-polymérase pour un milieu réactionnel de 50 µl.

13. Application du procédé selon l'une quelconque des revendications 10 à 12 au diagnostic *in vitro* de l'infection d'un individu par un virus du type HIV-1 et/ou HIV-2, ou d'un animal par au moins l'un des trois virus (HIV-1, HIV-2, SIV).

14. Kit pour la mise en oeuvre d'un procédé selon l'une des revendications 10 à 12 comprenant :
i) au moins une amorce oligonucléotidique ou un couple d'amorces oligonucléotidiques selon l'une quelconque des revendications 1 à 9,
ii) des réactifs appropriés à la mise en oeuvre du cycle d'opérations d'amplification, notamment de l'ADN polymérase, quatre nucléotides triphosphate différents, et le tampon 10X buffer de composition Tris-HCl, pH 8,9 : 50 mM ; (NH₄)₂ SO₄: 15 mM ; MgCl₂ : 5mM β-mercapto-éthanol : 10 mM ; gélatine : 0,25 mg/ml (en concentration finale d'utilisation),
iii) une (ou plusieurs) sonde, pouvant être marquée, capable de s'hybrider avec la (ou les) séquence d'acide nucléique amplifiée à détecter.

15. Utilisation d'au moins une amorce oligonucléotidique ou d'un couple d'amorces oligonucléotidiques selon l'une quelconque des revendications 1 à 9 pour l'amplification génique de séquences nucléiques du gène *pol* de virus du type HIV-1 et/ou HIV-2 et/ou SIV.

## Claims

1. An oligonucleotide, from 15 to 30 nucleotides, **characterized in that** its sequence consists of:
i) a specific sequence of the pol gene, which is capable of hybridizing at a temperature of 60°C ± 1°C with genomes of the HIV-1, Bru, HIV-1 Mal, HIV-1 Eli, HIV-1 Rod and SIV Mac viruses; or
ii) a complementary sequence of a sequence as defined in i).

2. An oligonucleotide, from 15 to 30 nucleotides, **characterized in that** its nucleotide sequence is modified with respect to the sequence of the oligonucleotide according to claim 1 and retains hybridization properties with the genomes of the HIV-1 Bru, HIV-1 Mal, HIV-1 Eli, HIV-2 Rod and SIV Mac viruses at a temperature of 60°C ± 1°C.

3. An oligonucleotide according to claim 2, wherein the 5 bases of each end are conserved with respect to those of the oligonucleotide primer described in claim 1, and which comprises in its median portion modifications with respect to the sequence of the oligonucleotide primer according to claim 1.

4. A specific oligonucleotide of the pol gene according to claim 1, selected from:
- 5'-TAA AGC CAG GAA TGG ATG GCC CAA-3',
- 5'-TAA AGC CAG GAA TGG ATG GAC CAA-3',
- 5'-TGG ACT GTC AAT GAC ATA CAGAA-3`,
- 5'-TGG ACT GTC AAT GAT ATA CAGAA-3',
- 5'-CAT GGG TAC CAG CAC ACAAAG G-3',
- 5'-TGG AAA GGT GAA GGG GCA GT-3',
- 5'-TGG AAA GGT GAAGGA GCA GT-3',
- 3'-TTG GGC CAT CCA TTC CTG GCT TTA-5',
- 3'-TTG GTC CAT CCA TTC CTG GCT TTA-5'.
- 3'-TTC TGT ATG TCA TTG ACA GTC CA-5'.
- 3'-TTC TGT ATG TCA TTG ACT GTC CA.5',
- 3'-CCT TTG TGT GCT GGT ACC CAT G-5',
- 3'-ACT GCC CCT TCA CCT TTC CA-5',
- 3'-ACT GCC CCT TCT CCT TTC CA-5'.
- 3'-ACT GCC CCT TCC CCT TTC CA-5'.

5. A pair of oligonucleotide primers, for carrying out gene amplification of the pol gene of a type HIV-1, HIV-2 and SIV virus, the primers each consisting of:
i) a specific sequence of the pol gene, from 15 to 30 nucleotides, and capable of hybridizing at a temperature of 60°C ± 1°C with the genomes of the HIV-1 Bru, HIV-1 Mal, HIV-1 Eli, HIV-2 Rod and SIV Mac viruses;
ii) a complementary sequence of a sequence as defined in i).

6. A pair of primers, **characterized in that** the sequence of at least one oligonucleotide primer of a pair of primers according to claim 5 is modified with respect to the sequence of the pol gene of the HIV-1 Bru, HIV-1 Mal, HIV-1 Eli, HIV-2 Rod and SIV Mac viruses, and retains hybridization properties with the genomes of the HIV-1 Bru, HIV-1 Mal, HIV-1 Eli, HIV-2 Rod and SIV Mac viruses at a temperature of 60°C ± 1°C.

7. A pair of primers according to claim 5, for carrying out gene amplification of the pol gene of a HIV-1, HIV-2 and SIV type virus, the primers consisting of:
i. at least one mixture selected from the following group of mixtures of sense sequences:
Mmy29: mixture constituted by the sequences
5'-TAA AGC CAG GAA TGG ATG GCC CAA-3'
5'-TAA AGC CAG GAA TGG ATG GAC CAA-3';
MMy30: mixture constituted by the sequences
5'-TGG ACT GTC AAT GAC ATA CAG AA-3'
5'- TGG ACT GTC AAT GAT ATA CAG AA -3';
MMy31: mixture constituted by the sequence
5'-CAT GGG TAC CAG CAC ACA AAG G-3';
MMy32: mixture constituted by the sequences
5'-TGG AAA GGT GAA GGG GCA GT-3'
5'- TGG AAA GGT GAA GGA GCA GT-3'; and
ii) at least one mixture selected from the following group of mixtures of antisense sequences:
MMy29bis: mixture constituted by the sequences
3'-TTG GGC CAT CCA TTC CTG GCT TTA-5'
3'- TTG GTC CAT CCA TTC CTG GCT TTA -5';
MMy30bis: mixture constituted by the sequences
3'-TTC TGT ATG TCA TTG ACA GTC CAT-5'
3'- TTC TGT ATG TCA TTG ACT GTC CA -5';
MMy31bis: mixture constituted by the sequence
3'-CCT TTG TGT GCT GGT ACC CAT G-5';
MMy32bis: mixture constituted by the sequences
3'-ACT GCC CCT TCA CCT TTC CA-5'
3'-ACT GCC CCT TCT CCT TTC CA -5'
3'-ACT GCC CCT TCC CCT TTC CA -5'.

8. A pair of primers according to claim 7, for carrying out gene amplification of the pol gene of a type HIV-1, HIV-2 and SIV virus, consisting of:
i) Mmy29-Mmy30bis
5'-TAA AGC CAG GAA TGG ATG GCC CAA-3'
5'-TAA AGC CAG GAA TGG ATG GAC CAA-3'
3'-TTC TGT ATG TCA TTG ACA GTC CA-5'
3'-TTC TGT ATG TCA TTG ACT GTC CA-5'
ii) Mmy30-Mmy31bis
5'-TGG ACT GTC AAT GAC ATA CAGAA-3'
5'-TGG ACT GTC AAT GAT ATA CAGAA-3'
3'-CCT TTG TGT GCT GGT ACC CAT G-5'
iii) Mmy31-Mmy32bis:
5'-CAT GGG TAC CAG CAC ACAAAG G-3'
3'-ACT GCC CCT TCA CCT TTC CAT-5'
3'-ACT GCC CCT TCT CCT TTC CA-5'
3'-ACT GCC CCT TCC CCT TTC CA-5'

9. An oligonucleotide or a pair of oligonucleotide primers according to any one of claims 1 to 8, capable of hybridization with the genomes of the HIV-1 Bru, HIV-1 Mal, HIV-1 Eli, HIV-2 Rod and SIV Mac viruses in a 10X buffer with the following composition: Tris-HCl, pH 8.9: 50 mM; (NH₄)₂SO₄: 15 mM; MgCl₂: 5 mM; β-mercapto-ethanol: 10 mM; gelatin: 0.25 mg/ml.

10. A method for gene amplification of nucleic sequences of the pol gene of type HIV-1 and/or HIV-2 and/or SIV virus, carried out on a biological sample, said method principally comprising the following steps:
a) a step for extracting nucleic acid to be detected belonging to the genome of a type HIV-1, HIV-2 or SIV virus which may be present in said biological sample and, if appropriate, a step for treating said nucleic acid with a reverse transcriptase if the nucleic acid is in the RNA form;
b) a cycle comprising the following steps:
• denaturing the double strand nucleic acid to be detected, resulting in the formation of a single strand nucleic acid;
• hybridizing each of the nucleic acid strands obtained during the preceding denaturing step with at least one oligonucleotide primer or pair of oligonucleotide primers according to any one of claims 1 to 9, by bringing said strands into contact with at least one pair of said oligonucleotide primers;
• forming, from the primers of the complementary DNA to the strands on which they are hybridized, in the presence of a DNA polymerase and four different nucleoside triphosphates (dNTP), resulting in the formation of a larger number of double strand nucleic acids to be detected than in the preceding denaturing step;
said cycle being repeated a predetermined number of times to obtain said nucleic sequence to be detected which may be present in the biological sample in a proportion which is sufficient to allow its detection;
c) a step for detecting the possible presence of a nucleic acid belonging to the genome of the type HIV-1 and/or HIV-2 and/or SIV virus in the biological sample.

11. A method according to claim 10, **characterized in that** the denaturing step is carried out in the presence of (an) oligonucleotide primer pair(s) according to one of claims 6 to 9.

12. A method according to claim 11, **characterized in that** it is carried out under the following conditions:
a) hybridization: bringing primers (1 µl of a 40 µmolar solution of each primer) into the presence of the DNA matrix (100 to 800 ng) for the first denaturing-reassociation step; heating for 10 minutes at 100°C then immersing the tubes containing said mixture of DNA-matrix and primers in water containing ice; the primers being used in a final concentration in the following amplification step of 0.8 µM a piece in the subsequent amplification step;
b) amplification: adding the four dNTPs to the above medium each in an amount of 0.5 µmolar in final solution (50 µl), and one unit of Taq polymerase for a reaction medium of 50 µl.

13. Application of the method according to any one of claims 10 to 12 to in vitro diagnosis of infection of an individual with a type HIV-1 and/or HIV-2 virus or of an animal with at least one of the three viruses (HIV-1, HIV-2, SIV).

14. A kit for carrying out the method according to one of claims 10 to 12, comprising:
i) at least one oligonucleotide primer or a pair of oligonucleotide primers according to any one of claims 1 to 9;
ii) reagents appropriate for carrying out the cycle of amplification operations, in particular DNA polymerase, four different triphosphate nucleotides, and the 10X buffer with composition Tris-HCl, pH 8.9: 50 mM; (NH₄)₂SO₄: 15 mM; MgCl₂: 5 mM; β-mercapto-ethanol: 10 mM; gelatin: 0.25 mg/ml, as a final concentration for use;
iii) one (or more) probes, which may be labeled, capable of hybridization with the amplified nucleic acid sequence(s) to be detected.

15. Use of at least one oligonucleotide primer or pair of oligonucleotide primers according to any one of claims 1 to 9, for the gene amplification of nucleic sequences of the pol gene of the type HIV-1 and/or HIV-2 and/or SIV virus.

## Patentansprüche

1. Oligonukleotid, von 15 bis 30 Nukleotiden, **dadurch gekennzeichnet, dass** seine Sequenz besteht aus:
i) einer spezifischen Sequenz des Gens po/, und geeignet ist, bei einer Temperatur von 60°C ± 1°C an die Genome der Viren HIV-1 Bru, HIV-1 Mal, HIV-1.Eli, HIV-2 Rod und SIV Mac zu hybridisieren, oder
ii) einer Komplementärsequenz einer Sequenz, wie sie unter i) definiert ist.

2. Oligonukleotid mit 15 bis 30 Nukleotiden, **dadurch gekennzeichnet, dass** seine Nukleotidsequenz modifiziert ist bezogen auf die Oligonukleotidsequenz gemäß Anspruch 1, und die Hybridiserungseigenschaften an die Genome der Viren HIV-1 Bru, HIV-1 Mal, HIV-1 Eli, HIV-2 Rod und SIV Mac bei einer Temperatur von 60°C ± 1°C behält.

3. Oligonukleotid gemäß Anspruch 2, bei dem die 5 Basen von jedem Ende konserviert sind, bezogen auf diejenigen eines Oligonukleotidstarters wie er in Anspruch 1 beschrieben ist, und das in seinem mittleren Teil Modifikationen bezogen auf die Sequenz eines Oligonukleotidstarters gemäß Anspruch 1 aufweist.

4. Spezifisches Oligonukleotid des Gens *pol* gemäß Anspruch 1, ausgewählt aus:
5'-TAA AGC CAG GAA TGG ATG GCC CAA-3'
5'-TAA AGC CAG GAA TGG ATG GAC CAA-3'
5'-TGG ACT GTC AAT GAC ATA CAG AA-3'
5'-TGG ACT GTC AAT GAT ATA CAG AA-3'
5'-CAT'GGG TAC CAG CAC ACA AAG G-3'
5'-TGG ACA GGT GAA GGG GCA GT-3'
5'-TGG AAA GGT GAA GGA GCA GT-3'
3'-TTG GGC CAT CCA TTC CTG GCT TTC-5'
3'-TTG GTC CAT CCA TTC CTG GCT TTA-5'
3'-TTC TGT ATG TCA TTG ACA GTC CA-5'
3'-TTC TGT ATG TCA TTG ACT GTC CAT-5'
3'-CCT TTG TGT GCT GGT ACC CAT G-5'
3'-ACT GCC CCT TCA CCT TTC CA-5'
3'-ACT GCC CCT TCT CCT TTC CA-5', und
3'-ACT GCC CCT TCC CCT TTC CA-5'

5. Oligonukleotidstarterpaar zur Durchführung einer Genamplifikation des Gens *pol* von Virus vom Typ HIV-1, HIV-2 und SIV, wobei jeder der Starter besteht aus:
i) einer spezifischen Sequenz des Gens *pol*, von 15 bis 30 Nukleotiden, und geeignet ist, bei einer Temperatur von 60°C ± 1°C an die Genome der Viren HIV-1 Bru, HIV-1 Mal, HIV-1 Eli, HIV-2 Rod und SIV Mac zu hybridisieren,
ii) einer Komplementärsequenz einer Sequenz, wie sie unter i) definiert ist.

6. Starterpaar, **dadurch gekennzeichnet, dass** die Sequenz von mindestens einem Oligonukleotidstarter von einem Starterpaar gemäß Anspruch 5 modifiziert ist bezogen auf die Sequenz des Gens *pol* der Viren HIV-1 Bru, HIV-1 Mal, HIV-1 Eli, HIV-2 Rod und SIV Mac, und die Hybridisierungseigenschaften an die Genome der Viren HIV-1 Bru, HIV-1 Mal, HIV-1 Eli, HIV-2 Rod und SIV Mac bei einer Temperatur von 60°C ± 1°C behält.

7. Starterpaar gemäß Anspruch 5 zur Durchführung einer Genamplifikation des Gens *pol* von Virus vom Typ HIV-1, HIV-2 oder SIV, wobei die Starter bestehen aus:
i. mindestens einer Mischung, die ausgewählt ist aus der folgenden Gruppe von Mischungen von Sense-Sequenzen:
Mmy29: Mischung, die gebildet ist aus den Sequenzen
5'-TAA AGC CAG GAA TGG ATG GCC CAA-3'
5'-TAA AGC CAG GAA TGG ATG GAC CAA-3'
Mmy30: Mischung, die gebildet ist aus den Sequenzen
5'-TGG ACT GTC AAT GAC ATA CAG AA-3'
5'-TGG ACT GTC AAT GAT ATA CAG AA-3'
Mmy31: Mischung, die gebildet ist aus der Sequenz
5'-CAT GGG TAC CAG CAC ACA AAG G-3'
Mmy32: Mischung, die gebildet ist aus den Sequenzen
5'-TGG AAA GGT GAA GGG GCA GT-3'
5'-TGG AAA GGT GAA GGA GCA GT-3', und
ii. mindestens einer Mischung, die ausgewählt ist aus der folgenden Gruppe von Mischungen von Antisense-Sequenzen:
Mmy29bis: Mischung, die gebildet ist aus den Sequenzen
3'-TTG GGC CAT CCA TTC CTG GCT TTA-5'
3'-TTG GTC CAT CCA TTC CTG GCT TTA-5'
Mmy30bis: Mischung, die gebildet ist aus den Sequenzen
3'-TTC TGT ATG TCA TTG ACA GTC CA-5'
3'-TTC TGT ATG TCA TTG ACT GTC CA-5'
Mmy31bis: Mischung, die gebildet ist aus der Sequenz
3'-CCT TTG TGT GCT GGT ACC CAT G-5'
Mmy32bis: Mischung, die gebildet ist aus den Sequenzen
3'-ACT GCC CCT TCA CCT TTC CA-5'
3'-ACT GCC CCT TCT CCT TTC CA-5'
3'-ACT GCC CCT TCC CCT TTC CA-5'.

8. Starterpaar gemäß Anspruch 7 zur Durchführung einer Genamplifikation des Gens *pol* von Virus vom Typ HIV-1, HIV-2 und SIV, bestehend aus:
i) Mmy29-Mmy30bis
5'-TAA AGC CAG GAA TGG ATG GCC CAA-3'
5'-TAA AGC CAG GAA TGG ATG GAC CAA-3'
3'-TTC TGT ATG TCA TTG ACA GTC CA-5'
3'-TTC TGT ATG TCA TTG ACT GTC CA-5'
ii) Mmy30-Mmy31bis
5'-TGG ACT GTC AAT GAC ATA CAG AA-3'
5'-TGG ACT GTC AAT GAT ATA CAG AA-3'
3'-CCT TTG TGT GCT GGT ACC CAT G-5'
iii) Mmy31-Mmy32bis:
5'-CAT GGG TAC CAG CAC ACA AAG G-3'
3'-ACT GCC CCT TCA CCT TTC CA-5'
3'-ACT GCC CCT TCT CCT TTC CA-5'
3'-ACT GCC CCT TCC CCT TTC CA-5'.

9. Oligonukleotid oder Oligonukleotidstarterpaar gemäß einem der Ansprüche 1 bis 8, das geeignet ist, an die Genome der Viren HIV-1 Bru, HIV-1 Mal, HIV-1 Eli, HIV-2 Rod und SIV Mac in einem Puffer 10 x Puffer der Zusammensetzung Tris-HCl, pH 8,9: 50 mM; (NH₄)₂SO₄: 15 mM; MgCl₂: 5 mM; β-Mercaptoethanol: 10 mM; Gelatine: 0,25 mg/ml zu hybridisieren.

10. Genamplifikationsverfahren von Nukleinsäuresequenzen des Gens *pol* von Virus vom Typ HIV-1 und/oder HIV-2 und/oder SIV, durchgeführt anhand einer biologischen Probe, wobei dieses Verfahren hauptsächlich die folgenden Schritte umfasst:
a) einen Schritt der Extraktion von zu detektierender Nukleinsäure, die zum Genom von Virus vom Typ HIV-1, HIV-2 oder SIV gehört, der möglicherweise in der oben erwähnten biologischen Probe vorhanden ist, und gegebenenfalls einen Schritt der Behandlung dieser Nukleinsäure mit Hilfe einer reversen Transkriptase, wenn diese Nukleinsäure in Form von RNA vorliegt,
b) einen Zyklus, der folgende Schritte umfasst:
- Denaturierung der zu detektierenden Doppelstrang-Nukleinsäure, was zur Bildung einer Einzelstrang-Nukleinsäure führt,
- Hybridisierung von jedem der Nukleinsäurestränge, die bei dem vorhergehenden Denaturierungsschritt erhalten wurden, mit mindestens einem Oligonukleotidstarter oder einem Oligonukleotidstarterpaar gemäß einem der Ansprüche 1 bis 9 durch Inkontaktbringen der oben erwähnten Stränge mit mindestens einem oben erwähnten Oligonukleotidstarter,
- Bildung, ausgehend von DNA-Startern, die zu den Strängen komplementär sind, auf die sie hybridisiert sind in Gegenwart einer DNA-Polymerase und vier unterschiedlichen Nukleosidtriphosphaten (dNTP), was zur Bildung einer größeren Anzahl von zu detektierenden Doppelstrang-Nukleinsäuren führt als beim vorhergehenden Schritt der Denaturierung,
wobei dieser Zyklus eine bestimmte Anzahl von Malen wiederholt wird, um die möglicherweise in der biologischen Probe vorliegende, zu detektierende Nukleinsäuresequenz in einem ausreichenden Ausmaß zu erhalten, um deren Detektion zu ermöglichen,
c) einen Schritt des Nachweises eines möglichen Vorhandenseins von Nukleinsäure, die zum Genom von Virus vom Typ HIV-1 und/oder HIV-2 und/oder SIV gehört, in der biologischen Probe.

11. Verfahren gemäß Anspruch 10, **dadurch gekennzeichnet, dass** der Denaturierungsschritt in Gegenwart des (oder der) Oligonukleotidstarterpaars (Oligonukleotidstarterpaare) gemäß einem der Ansprüche 6 bis 9 durchgeführt wird.

12. Verfahren gemäß Anspruch 11, **dadurch gekennzeichnet, dass** es unter folgenden Bedingungen durchgeführt wird:
a) Hybridisierung: die Oligonukleotidstarter (1 µl einer Lösung von 40 µmolar von jedem der Starter) werden für den ersten Schritt der Denaturierung-Reassoziation mit DNA-Matrix (100 bis 300 ng) zusammengebracht; es wird 10 Minuten lang bei 100°C erhitzt, und daraufhin werden die Gefäße, die diese Mischung aus DNA-Matrix und Startern enthalten, in Eis-haltiges Wasser getaucht. Die Starter sollen im folgenden Amplifikationsschritt jeweils in einer Endkonzentration von 0,8 µM verwendet werden,
b) Amplifikation: man fügt dem vorher beschriebenen Medium die 4 dNTPs, wobei jedes mit 0,5 µmolar in der Endlösung (50 µl) verwendet wird, und eine Einheit von Taq-Polymerase für ein Reaktionsmedium von 50 µl hinzu.

13. Verwendung des Verfahrens gemäß einem der Ansprüche 10 bis 12 zur *in vitro-*Diagnose der Infektion eines Individuums mit einem Virus vom Typ HIV-1 und/oder HIV-2, oder eines Tieres mit mindestens einem der drei Viren (HIV-1, HIV-2, SIV).

14. Kit zur Durchführung eines Verfahrens gemäß einem der Ansprüche 10 bis 12, umfassend:
i) mindestens einen Oligonukleotidstarter oder ein Oligonukleotidstarterpaar gemäß einem der Ansprüche 1 bis 9,
ii) geeignete Reagentien zur Durchführung des Zyklus der Arbeitsgänge der Amplifikation, insbesondere DNA-Polymerase, vier unterschiedliche Nukleotidtriphosphate und den Puffer 10 x Puffer der Zusammensetzung Tris-HCl, pH 8,9: 50 mM; (NH₄)₂SO₄: 15 mM; MgCl₂: 5 mM; β-Mercaptoethanol: 10 mM; Gelatine: 0,25 mg/ml (Verwendungsendkonzentration),
iii) eine (oder mehrere) Sonde(n), die markiert sein kann (können), die in der Lage ist (sind), an die zu detektierende(n), amplifizierte(n) Nukleinsäuresequenz(en) zu hybridisieren.

15. Verwendung von mindestens einem Oligonukleotidstarter oder einem Oligonukleotidstarterpaar gemäß einem der Ansprüche 1 bis 9 zur Genamplifikation von Nukleinsäuresequenzen des Gens *pol* von Virus von Typ HIV-1 und/oder HIV-2 und/oder SIV.
